# EUROPÄISCHE PATENTANMELDUNG

(11) **EP 3 590 916 A1**
(43) Veröffentlichungstag der Anmeldung: **08.01.2020**
(21) Anmeldenummer: 18181804.8
(22) Anmeldetag: 05.07.2018
(51) Int. Cl.: C07C 68/02, C07C 69/96

(54) **VERFAHREN ZUR HERSTELLUNG VON DIALKYLDICARBONATEN MIT QUATERNÄREN AMMONIUMKATALYSATOREN**

(71) Anmelder: LANXESS Deutschland GmbH, 50569 Köln (DE)
(72) Erfinder: HOFMANN, Christoph, 51061 Köln (DE)

(57) **Zusammenfassung**

Die vorliegende Erfindung betrifft ein Verfahren zur Herstellung von Dialkyldicarbonaten aus den entsprechenden Chlorameisensäurealkylestern unter Verwendung von ausgewählten quaternären Ammoniumkatalysatoren.

## Beschreibung

Die vorliegende Erfindung betrifft ein Verfahren zur Herstellung von Dialkyldicarbonaten aus den entsprechenden Chlorameisensäurealkylestern unter Verwendung von ausgewählten quaternären Ammoniumkatalysatoren.

Dialkyldicarbonate finden z.B. als Katalysatoren zur Oxidation von sterisch anspruchsvollen Aminen, als Bestandteile von Elektrolytflüssigkeiten oder als Bestandteile von antimikrobiellen Reagenzien Verwendung. Dialkyldicarbonate werden in der Literatur auch als Dialkylpyrocarbonate bezeichnet.

In DE-A 1 418 849 werden tertiäre Amine als besonders geeignete Katalysatoren für die Herstellung von Säurederivaten beschrieben, deren tertiäre Stickstoffatome sterisch nicht gehindert sind, ausgenommen tertiäre Amine, die als Substituenten am Stickstoff ebensolche ω-Hydroxyalkyl-, ω-Hydroxyalkyläther- oder ω-Hydroxyalkylpolyäthergruppe tragen. Neben Triethylamin und Tri-n-butylamin kommen hier deshalb Amine zum Einsatz, die mindestens eine Methylgruppe am Stickstoff tragen, wie z.B. N-Methyl-di-n-stearylamin. Diese Katalysatoren besitzen aber u.a. den Nachteil, dass sie nicht nur die Bildung, sondern auch die Zersetzung des Produktes katalysieren, was zu einer Verringerung der Ausbeute führt. Zudem sind einige dieser Katalysatoren toxisch, werden im Abwasser schlecht abgebaut und lassen sich vom Reaktionsgemisch aufgrund eigener Zersetzungen während der Reaktion schlecht abtrennen.

Aus DE-B 1210 853 ist bekannt, Kohlensäure- oder Carbonsäurehalogenide mit organischen Hydroxylverbindungen oder deren Alkali- oder Erdalkalisalzen und organischen, mit Wasser nicht mischbaren Lösungsmitteln und mindestens äquivalenten Mengen an Alkali- oder Erdalkalihydroxiden oder -carbonaten in einem zweiphasigen System, sowie unter Verwendung katalytischer Mengen an tertiären Aminen oder ihren Quaternierungsprodukten, umzusetzen, wobei als Amine oder deren Quaternierungsprodukte solche eingesetzt werden, die mindestens eine an Stickstoff gebundene ω-Hydroxyalkyl-, ω-Hydroxyalkyläther- oder ω-Hydroxyalkylpolyäthergruppe tragen.

Um die verbesserte Wirkungsweise der aus der DE-B 1210853 bekannten Amine und Quaternisierungsprodukte zu belegen, ist aus der DE-B 1210853 bekannt, dass quaternierte Ammoniumverbindungen, wie z.B. Triethylbenzylammoniumchlorid als Katalysatoren eingesetzt werden können. Gemäß der DE-B 1210853 zeigen aber gerade diese quaternierten Ammoniumverbindungen sehr geringe Ausbeuten und sind daher für die Verwendung in einem Verfahren zur Herstellung von Dialkyldicarbonaten ungeeignet.

Es bestand daher weiterhin Bedarf an einem Herstellungsverfahren, welches das Zielprodukt in hoher Ausbeute liefert.

Überraschenderweise wurde jetzt gefunden, dass Dialkyldicarbonate aus Halogenameisensäurealkylestern durch Umsetzung mit Alkali- oder Erdalkalihydroxiden oder -carbonaten besonders vorteilhaft erhalten werden können, wenn man als Katalysatoren spezielle quaternäre Ammoniumverbindungen der Formel (I) einsetzt. Diese zeichnen sich durch eine hohe katalytische Aktivität aus, ohne zersetzend auf das Endprodukt zu wirken und können leicht vom Produkt, z.B. durch Extraktion abgetrennt werden. Zudem ist die Ausbeute an Dialkyldicarbonate mit den Verbindungen der Formel (I) höher als mit herkömmlichen tertiären Aminen oder quaternären Ammoniumverbindungen, wie z.B. dem aus der DE-B 1210853 bekannten Triethylbenzylammoniumchlorid.

Gegenstand der vorliegenden Erfindung ist demnach ein Verfahren zur Herstellung von Dialkyldicarbonaten durch Umsetzung der entsprechenden Halogenameisensäurealkylester mit Alkali- oder Erdalkalihydroxiden und/oder -carbonaten in Gegenwart von mit Wasser nicht mischbaren organischen Lösungsmitteln und in Gegenwart eines Katalysators dadurch gekennzeichnet ist, dass als Katalysator mindestens eine Verbindung der Formel (I)

X[NR¹R²R³R⁴] (I)

worin
R¹ und R²= geradkettiges oder verzweigtes C₁-C₆-Alkyl,
R³ = geradkettiges oder verzweigtes C₁₀-C₂₂-Alkyl,
R⁴ = Phenyl oder Benzyl,
X = einwertiges Anion
wobei R¹ und R² jeweils unabhängig voneinander sind und gleich oder verschieden sein können, eingesetzt wird.

Bevorzugt werden bei der Durchführung des erfindungsgemäßen Verfahrens als Katalysator eine Verbindung der Formel (I) eingesetzt, worin R¹ und R² = Methyl, Ethyl, s-Propyl, n-Propyl, iso-Butyl, s-Butyl, n-Butyl oder t-Butyl ist und R³ = geradkettiges oder verzweigtes C₁₀-C₁₈-Alkyl.

X ist bevorzugt ein Halogenid, besonders bevorzugt ist X = Cl.

Besonders bevorzugt werden als Katalysatoren Verbindungen der Formel (I) mit R¹ und R² = Methyl und R³ = C₁₀-C₁₆-Alkyl eingesetzt.

In einer Ausführungsform des erfindungsgemäßen Verfahrens wird als Katalysator eine Verbindung der Formel (I) eingesetzt, bei dem R³ jeweils geradkettiges oder verzweigtes Dodecanyl, Undecanyl, Tridecanyl, Tetradecanyl, Pentadecanyl, Hexadecanyl, Heptadecanyl, Octadecanyl, Nonadecanyl, Eicosanyl, Icosanyl, Heneicosanyl oder Dodoconyl bedeutet. Bevorzugt ist R³ = Dodecanyl, Tetradecanyl, Hexadecanyl oder Octadecanyl.

In einer weiteren Ausführungsform des erfindungsgemäßen Verfahrens wird als Katalysator eine Verbindung der Formel (I) bei der R¹ und R² = Methyl und R³ = Dodecanyl, Tetradecanyl und Hexadecanyl ist.

In einer weiteren Ausführungsform der Erfindung ist R⁴ = Benzyl.

Bei der Durchführung des erfindungsgemäßen Verfahrens können auch beliebige Mischungen der Katalysatoren eingesetzt werden.

In einer weiteren bevorzugten Ausführungsform werden als Katalysatoren Mischungen von Verbindungen der Formel (I) eingesetzt, die verschiedene Reste R³ mit geradkettigem oder verzweigtem C₁₀-, C₁₁-, C₁₂-, C₁₃-, C₁₄-, C₁₅-, C₁₆-, C₁₇- oder C₁₈-Alkyl aufweisen. Noch weiter bevorzugt werden Mischungen von Verbindungen der Formel (I) eingesetzt, die verschiedene Reste R³ mit geradkettigem oder verzweigtem C₁₂-, C₁₄- und C₁₆-Alkyl beinhalten und bei denen R¹ und R² = Methyl und R⁴ = Benzyl ist.

In einer weiteren bevorzugten Ausführungsform werden als Katalysatoren Mischungen der Verbindungen der Formel (I) eingesetzt, die verschieden Reste R³ mit geradkettigem oder verzweigtem C₁₂-, C₁₄- und C₁₆-Alkyl und R¹ und R² = Methyl und R⁴ = Benzyl aufweisen und bei denen der Gehalt der Verbindung mit R³ = C₁₂-Alkyl von 60 Gew.% und 80 Gew.% und der Gehalt der Verbindung mit R³ = C₁₄-Alkyl von 19 Gew. % und 30 Gew. % und der Gehalt der Verbindung mit R³ = C₁₆-Alkyl von 1 Gew. % und 10 Gew.% bezogen auf die Menge an eingesetzter Verbindung der Formel (I) ist.

In einer weiteren bevorzugten Ausführungsform können als Katalysatoren Mischungen der Verbindungen der Formel (I) eingesetzt werden, bei denen die Reste R¹, R², R³ und R⁴ im Rahmen der obigen Offenbarung in beliebiger Art kombiniert werden.

Die zur Herstellung der Katalysatoren üblicherweise verwendeten tertiären Amine und Alkylierungsmittel, wie beispielsweise Methylchlorid oder Dodecylbromid, sind kommerziell erhältlich. Quartäre Ammoniumverbindungen werden durch Umsetzung tertiärer Amine mit Alkylierungsmitteln, wie z. B. Methylchlorid, Benzylchlorid, Dimethylsulfat, Dodecylbromid, oder Ethylenoxid hergestellt. Ihre Herstellungsverfahren sind ferner dem Fachmann bekannt. Quartäre Ammoniumverbindungen als Katalysatoren sind kommerziell verfügbar. Alkyldimethylbenzylammonium chloride wird unter dem Namen Barquat DM-50, von der Fa. Lonza vertrieben.

Vorzugsweise dient das erfindungsgemäße Verfahren zur Herstellung von Dialkyldicarbonaten der Formel (II) 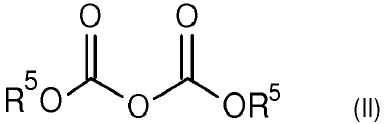 worin
- R⁵: für geradkettiges oder verzweigtes C₁-C₂₀-Alkyl steht,
durch Umsetzung von Halogenameisensäurealkylester der Formel (III) 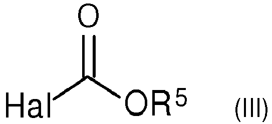 worin
- Hal: für Halogen, bevorzugt für F, Cl, Br, I, insbesondere für Chlor steht, und
- R⁵: für geradkettiges oder verzweigtes C₁-C₂₀-Alkyl steht.

In den Formeln (II) und (III) steht R⁵ vorzugsweise für geradkettiges oder verzweigtes C₁-C₈-Alkyl, besonders bevorzugt für einen Rest -CH-R⁶R⁷, wobei R⁶ und R⁷ unabhängig voneinander für H oder für geradkettiges oder verzweigtes C₁-C₇-Alkyl stehen. Insbesondere steht R⁵ für Methyl, Ethyl, n-Propyl, iso-Propyl, n-Butyl oder iso-Butyl. Insbesondere bevorzugt steht R⁵ für Methyl, so dass Dimethyldicarbonat als Verbindung der Formel (II) erhalten wird.

Als Alkali- oder Erdalkalihydroxide oder -carbonate kommen beispielsweise LiOH, NaOH, KOH, LiCO₃, Na₂CO₃, K₂CO₃ in Frage. Vorzugsweise werden Alkalihydroxide verwendet, wie Natrium- und Kaliumhydroxid, die vorzugsweise in Form von wässrigen Lösungen zum Einsatz gelangen. Beispielsweise kann man 1 bis 50 gew.-%ige wässrige Alkalihydroxidlösungen verwenden. Bevorzugt sind 5 bis 35 gew.-%ige Lösungen, besonders bevorzugt 10 bis 25 gew.%ige Lösungen. Die Alkali- oder Erdalkalihydroxide oder -carbonate werden vorzugsweise in Mengen von 80 bis 120 Mol-% bezogen auf eingesetzten Halogenameisensäureester eingesetzt. Besonders bevorzugt liegt diese Menge im Bereich von 90 bis 115 Mol-%, ganz besonders bevorzugt im Bereich von 95 bis 115 Mol-%.

Als mit Wasser nicht mischbare organische Lösungsmittel kommen beispielsweise aliphatische und aromatische Kohlenwasserstoffe, halogenierte Kohlenwasserstoffe, mit Wasser nicht mischbare Ether oder Ester sowie Dialkylcarbonate in Frage. Bevorzugt sind Cyclohexan, Toluol, Xylol, Methylenchlorid und Diethylether, insbesondere Toluol und Methylenchlorid. Ganz besonders bevorzugt wird Toluol eingesetzt.

Das mit Wasser nicht mischbare organische Lösungsmittel kann man beispielsweise in Mengen von 20 bis 90 Gew.-%, bevorzugt von 30 bis 80 Gew.-%, besonders bevorzugt von 40 bis 80 Gew.-% bezogen auf den Halogenameisensäureester der Formel (III) einsetzen.

Der Katalysator der Formel (I) wird im Allgemeinen bezogen auf Halogenameisensäureester der Formel (III), in einer Menge von 0,001 bis 5 Mol-%, vorzugsweise von 0,005 bis 5 Mol-% eingesetzt. Noch weiter bevorzugt wird der Katalysator der Formel (I) in einer Menge von 0,005 bis 2 Mol-% eingesetzt.

Das erfindungsgemäße Verfahren kann in einem Druckbereich von 1 bis 10 bar, vorzugsweise von 1 bis 1,5 bar durchgeführt werden.

Die Reaktionstemperatur kann beispielsweise zwischen -10°C und der Siedetemperatur (bei Normaldruck) des eingesetzten Halogenameisensäureesters liegen. Vorzugsweise liegt sie im Bereich 0 bis 50°C. Noch weiter bevorzugt liegt die Reaktionstemperatur zwischen 15°C und 25°C.

Es ist vorteilhaft, während der Durchführung des erfindungsgemäßen Verfahrens für eine gute Durchmischung zu sorgen, z.B. durch Einsatz von Rührwerken, Strömungsstörern oder Umwälzpumpen.

Das erfindungsgemäße Verfahren kann sowohl batchweise als auch kontinuierlich durchgeführt werden. Bei batchweiser Arbeitsweise wird die Umsetzung bevorzugt in einem Rührkessel durchgeführt. Die Reaktion ist hierbei je nach Größe des Ansatzes und der vorhandenen Kühlleistung im allgemeinen nach 10 Minuten bis 3 Stunden beendet.

Vorzugsweise wird das erfindungsgemäße Verfahren kontinuierlich unter Verwendung eines Rührkessels, einer Rührkesselkaskade oder eines Rohrreaktors durchgeführt. Hierbei beträgt die mittlere Verweilzeit im Reaktor in der Regel zwischen 1 und 60 Minuten, bevorzugt zwischen 6 und 45 Minuten und besonders bevorzugt zwischen 10 und 30 Minuten.

Nach der Durchführung des erfindungsgemäßen Verfahrens, gegebenenfalls nach Abkühlung trennt sich das Reaktionsgemisch in zwei Phasen auf. Die organische Phase enthält neben dem Lösungsmittel das hergestellte Dialkyldicarbonat und gegebenenfalls geringe Mengen nicht umgesetzten Halogenameisensäureester sowie den Katalysator. Die wässrige Phase enthält neben Wasser die gebildeten anorganischen Salze.

Aus der organischen Phase kann man das Reaktionsprodukt durch mehrstufige Destillation in hoher Reinheit gewinnen. Der Katalysator kann zuvor durch Extraktion abgetrennt werden und gegebenenfalls nach einer Aufreinigung wieder in dem erfindungsgemäßen Verfahren als Katalysator eingesetzt werden (Recyclisierung).

Es ist ein besonderer und überraschender Vorteil des erfindungsgemäßen Verfahrens, dass der Katalysator die Zersetzung der Dialkyldicarbonate nach der Reaktion praktisch nicht katalysiert wodurch die isolierte Ausbeute an Endprodukt im Vergleich zu herkömmlichen Verfahren höher ist. Die eingesetzten Katalysatoren sind den bisherigen bekannten Phasen-Transfer Katalysatoren zudem im Hinblick auf Abtrennung und Rückgewinnung deutlich überlegen.

Mit Hilfe der erfindungsgemäßen Katalysatoren der Verbindungen der Formel (I) können Dialkyldicarbonate in hohen Ausbeuten hergestellt werden.

### Beispiele

### Beispiel 1

In einem Reaktionsgefäß wird ein Gemisch aus 19,66 g (0,21 mol) Chlormethylameisensäureester (CAME) und 14,72 g Toluol vorgelegt und dann 0,81 g Alkyldimethylbenzylammonium chloride (0,0013 mol), Gemisch aus 70% C₁₂-, 26% C₁₄- und 4% C₁₆-Alkylresten bezogen auf die gesamte Menge des eingesetzten Alkyldimethylbenzylammonium chloride, (Barquat DM 50 (Lonza, Basel) enthält 50 % Wasser) und 64,81 g einer ca. 14%iger NaOH (0,227 mol) eingeleitet.

Dieses wird unter intensivem Rühren bei 18°C zur Reaktion gebracht. Nach ca. 20 min wurden die Phasen getrennt. Die Rohausbeute liegt bei ca. 92% bezogen auf die eingesetzte Menge CAME.

### Vergleichsbeispiel 1 (mit einem quaternären Ammoniumsalz als Katalysator, wie z.B. aus Beispiel 6 i) der DE-B 1210 853 bekannt)

In einem Reaktionsgefäß wird ein Gemisch aus 18,87 g (0,2 mol) CAME und 13,98 g Toluol vorgelegt und dann 1,35 g (0,0015 mol) Bardac 2240 (40% in Wasser (N,N-Didecyl-N,N-dimethylammoniumchlorid)) und 65,80 g (0,23 mol) einer ca. 14%iger NaOH eingeleitet.

Dieses wird unter intensivem Rühren bei 24°C zur Reaktion gebracht. Nach ca. 20 min wurden die Phasen getrennt

Die Rohausbeute liegt bei ca. 57% bezogen auf die eingesetzte Menge CAME.

## Patentansprüche

1. Verfahren zur Herstellung von Dialkyldicarbonaten durch Umsetzung der entsprechenden Halogenameisensäurealkylester mit mindestens einem Alkalihydroxid, Erdalkalihydroxid und/oder -carbonat in Gegenwart von mindestens einem, mit Wasser nicht mischbaren organischen Lösungsmittel und in Gegenwart eines Katalysators **dadurch gekennzeichnet, dass** als Katalysator mindestens eine Verbindung der Formel (I)
X[NR¹R²R³R⁴] (I)
worin
R¹ und R² = geradkettiges oder verzweigtes C₁-C₆-Alkyl,
R³ = geradkettiges oder verzweigtes C₁₀-C₂₂-Alkyl,
R⁴ = Phenyl oder Benzyl,
X = einwertiges Anion
wobei R¹ und R² jeweils unabhängig voneinander sind und gleich oder verschieden sein können, eingesetzt wird.

2. Verfahren gemäß Anspruch 1, **dadurch gekennzeichnet dass** als Katalysator mindestens eine Verbindung der Formel (I) gemäß Anspruch 1 eingesetzt wird, worin R³ jeweils geradkettiges oder verzweigtes Dodecanyl, Undecanyl, Tetradecanyl, Hexadecanyl, Heptadecanyl, Octadecanyl, Nonadecanyl, Eicosanyl, Icosanyl, Heneicosanyl oder Dodoconyl ist.

3. Verfahren gemäß Anspruch 1 oder 2, **dadurch gekennzeichnet dass** als Katalysator mindestens eine Verbindung der Formel (I) gemäß Anspruch 1 eingesetzt wird, worin R¹ und R² = Methyl, Ethyl, Propyl oder Butyl ist und R³ = geradkettiges oder verzweigtes C₁₀-C₁₈ - Alkyl.

4. Verfahren gemäß wenigstens einem der Ansprüche 1 bis 3, **dadurch gekennzeichnet dass** als Katalysator mindestens eine Verbindung der Formel (I) gemäß Anspruch 1 eingesetzt wird, worin R⁴ = Benzyl.

5. Verfahren gemäß wenigstens einem der Ansprüche 1 bis 4, **dadurch gekennzeichnet dass** als Katalysator Mischungen von Verbindungen der Formel (I) eingesetzt werden, die verschiedene Reste R³ mit geradkettigem oder verzweigtem C₁₂-, C₁₄- und C₁₆-Alkyl beinhalten und bei denen R¹ und R² = Methyl und R⁴ = Benzyl ist.

6. Verfahren gemäß Anspruch 5, **dadurch gekennzeichnet dass** als Verbindungen der Formel (I) Mischungen eingesetzt werden, bei denen der Gehalt der Verbindung mit R³ = C₁₂-Alkyl, R¹ und R² = Methyl und R⁴ = Benzyl, von 60 Gew.% und 80 Gew.% und der Gehalt der Verbindung mit R³ = C₁₄-Alkyl, R¹ und R² = Methyl und R⁴ = Benzyl von 19 Gew. % und 30 Gew. % und der Gehalt der Verbindung mit R³ = C₁₆-Alkyl, R¹ und R² = Methyl und R⁴ = Benzyl von 1 Gew. % und 10 Gew.% bezogen auf die Menge an eingesetzter Verbindung der Formel (I) ist.

7. Verfahren gemäß wenigstens einem der Ansprüche 1 bis 6, **dadurch gekennzeichnet dass** als Katalysator eine Mischung der Verbindungen der Formel (I) eingesetzt wird, **dadurch gekennzeichnet, dass** die Katalysatoren verschiedene Reste R³ mit geradkettigem oder verzweigtem C₁₀-, C₁₁₋, C₁₂-, C₁₃-, C₁₄- C₁₅- C₁₆-, C₁₇-, C₁₈-Alkyl aufweisen.

8. Verfahren zur Herstellung von Dialkyldicarbonaten der Formel (II) 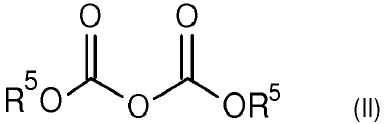 worin
R⁵ für geradkettiges oder verzweigtes C₁-C₂₀-Alkyl steht,
durch Umsetzung von Halogenameisensäurealkylester der Formel (III) 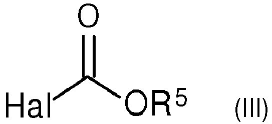 worin
Hal für Halogen, bevorzugt für F, Cl, Br, I, insbesondere für Chlor steht, und
R⁵ für geradkettiges oder verzweigtes C₁-C₂₀-Alkyl steht,
**dadurch gekennzeichnet, dass** die Umsetzung in Gegenwart mindestens einer quaternären Ammoniumverbindung der Formel (I)
X[NR¹R²R³R⁴] (I)
worin
R¹ und R²= geradkettiges oder verzweigtes C₁-C₆-Alkyl,
R³ = geradkettiges oder verzweigtes C₁₀-C₂₂-Alkyl,
R⁴ = Phenyl oder Benzyl,
X = einwertiges Anion
wobei R¹ und R² jeweils unabhängig voneinander sind und gleich oder verschieden sein können,
als Katalysator durchgeführt wird.

9. Verfahren gemäß wenigstens einem der Ansprüche 1 bis 8, **dadurch gekennzeichnet, dass** die Alkalihydroxide, Erdalkalihydroxide und/odercarbonate in Form wässriger Lösungen einsetzt werden.

10. Verfahren gemäß wenigstens einem der Ansprüchen 1 bis 9, **dadurch gekennzeichnet, dass** man mindestens ein mit Wasser nicht mischbares organisches Lösungsmittel aus der Reihe der aliphatischen und aromatischen Kohlenwasserstoffe, der chlorierten Kohlenwasserstoffe, der Dialkylcarbonate oder der mit Wasser nicht mischbaren Ether und Ester einsetzt.

11. Verfahren gemäß wenigstens einem der Ansprüche 1 bis 7, **dadurch gekennzeichnet, dass** die Menge an eingesetzten Verbindungen der Formel (I) 0,001 bis 5 Mol-%, bezogen auf Halogenameisensäureester, beträgt.

12. Verfahren gemäß wenigstens einem der Ansprüche 1 bis 7, **dadurch gekennzeichnet, dass** die Menge an eingesetzten Verbindungen der Formel (I) 0,005 bis 2 Mol-%, bezogen auf Halogenameisensäureester, beträgt.

13. Verfahren gemäß wenigstens einem der Ansprüche 1 bis 12, **dadurch gekennzeichnet, dass** die Reaktion bei einer Temperatur zwischen 15°C und 20 °C (bei Normaldruck) des eingesetzten Halogenameisensäureesters durchgeführt wird.

14. Verfahren gemäß wenigstens einem der Ansprüche 1 bis 13, **dadurch gekennzeichnet, dass** die Umsetzung in kontinuierlicher Arbeitsweise durchgeführt wird.

15. Verfahren gemäß wenigstens einem der Ansprüche 1 bis 14, **dadurch gekennzeichnet, dass** nach Beendigung der Umsetzung die Aufarbeitung des Reaktionsgemisches zur Abtrennung des Dialkyldicarbonats durch Phasentrennung und anschließender mehrstufiger Destillation der organischen Phase erfolgt.
